# EUROPEAN PATENT APPLICATION

(11) **EP 4 710 912 A1**
(43) Date of publication of application: **18.03.2026**
(21) Application number: 24199794.9
(22) Date of filing: 11.09.2024
(51) Int. Cl.: A61K 8/02, A61K 8/365, A61K 8/9789, A61K 31/191, A61K 36/736, A61Q 19/00, A61Q 19/10, A61P 17/00, A61P 17/04, A61P 17/06, A61P 17/08, A61P 17/10, A61P 17/12

(54) **PLANT-BASED COMPOSITION CONTAINING SHIKIMIC ACID WITH ENHANCED STABILITY IN BIOPHARMACEUTICAL FORMS FOR SELECTIVE MANAGEMENT OF HORMONAL SKIN STRESS**

(71) Applicant: SkyLab AG, 1066 Epalinges (CH)
(72) Inventor: Belous, Elena Yur'evna, 121309 MOSCOW (RU); Filatov, Viktor Andreevich, 119415 MOSCOW (RU); Iagafarova, Irina Evgenevna, Kiryu, Gunma-ken, 376-0052 (JP)
(74) Representative: Glawe, Delfs, Moll

(57) **Abstract**

The invention relates to a composition comprising shikimic acid and an extract of *Prunus Mume,* and its non-medical and medical use, in particular for use in the treatment of acne.

## Description

### FIELD OF THE INVENTION

The invention relates to the field of dermatology and specifically pertains to a plant-based composition for skin care of the body and scalp, designed to manage hormonal skin stress and to modulate epigenetically the 5α-reductase type I activity in skin cells, particularly in epidermal cells, sebocytes, and hair follicle cells. The invention relates to a biocomplex and its application in various perfumery and cosmetic products, comprising components A and B, where:
(A) Shikimic acid or its salts, shikimates, of plant origin;
(B) Extract of Japanese apricot *Prunus Mume,*
where the mass ratio of the components, in terms of active substances, is (0.10-1.0):(0.001-1.0) wt. % respectively.

The composition is intended for inclusion in perfumery and cosmetic products for body and scalp care, the application of which reduces the effects of hormonal stress induced by testosterone, cortisol, and steroid sex hormones, and epigenetically modulates the quantity and expression of 5α-reductase in various skin cells. The composition features enhanced solubility and stability due to the combination of shikimic acid and *Prunus Mume* extract, is of natural origin, exerts a safe effect on epidermal skin cells during prolonged contact, and can be used in the production of perfumery and cosmetic products, such as skin care patches, cleansers, lotions and tonics, face serums, cleansing and care masks, facial creams, and hair care products. The use of this complex contributes to the reduction of excessive 5α-reductase expression under hormonal stress caused by elevated levels of testosterone-related compounds, thereby managing the skin's emotional state and preventing hormonal changes, such as acne.

### BACKGROUND OF THE INVENTION

It is widely accepted that steroid hormones are synthesized in the adrenal cortex, ovaries, testes, and placenta. However, the skin can also be considered a steroidogenic organ, capable of producing glucocorticoids, androgens, and estrogens. Due to specific enzymes, androgen conversion into their more potent analogs also occurs in skin cells. Disruptions in hormone synthesis processes, elevated androgen levels, and decreased estrogen levels can lead to various skin manifestations, such as atopic dermatitis or seborrheic dermatitis. One of the main consequences of hormonal stress is an increase in sebum production, leading to oilier skin and a propensity for inflammation [Andrzej Slominski et al, Steroidogenesis in the skin: Implications for local immune functions // The Journal of Steroid Biochemistry and Molecular Biology, 2013, Volume 137].

Hormonal imbalances in the body can be caused by poor diet, stress, hormonal medication intake, and sleep problems. Nowadays, people often experience significant stress due to social and personal issues, which is detrimental to health. Stress causes weight loss, reduces energy, and leads to menstrual cycle problems that disrupt the hormonal cycle, as well as skin and hair issues, such as acne, psoriasis, and eczema. It has been tested and proven that stress induces the production of hormones like cortisol and weakens the immune system, directly exacerbating skin diseases. Under stress, the body depletes various essential vitamins and minerals, such as vitamin C, potassium, vitamin B, and magnesium, which are necessary for hormonal balance [Jusuf N. K., Putra I. B., Sutrisno A. R. Correlation between stress scale and serum substance p level in acne vulgaris // International Journal of General Medicine, 2021, 14]. Increased levels of 5-alpha reductase expression are observed in the body's tissues during hormonal stress [Sánchez P, Torres JM, Gavete P, Ortega E. Effects of swim stress on mRNA and protein levels of steroid 5alpha-reductase isozymes in prefrontal cortex of adult male rats. Neurochem Int., 2008, 52(3):426-31].

One of the manifestations of hormonal skin stress is acne. Acne is the most common chronic inflammatory disease of the pilosebaceous unit, primarily caused by increased sebum production, follicular hyperkeratinization, alteration of facial skin microbiota, and inflammation. Currently, acne is a prevalent condition among people of various age groups. Clinical experience shows that an estimated 85% of adults experience some form of acne during their lifetime G., Zaenglein A. L. Clinical Practice Acne Vulgaris // N ENGL J MED, 2018,14].

The disease usually begins during puberty, when sex hormones start being produced and sebum production increases. It is most commonly found in adolescents and young adults, with a subsequent progressive decline in prevalence with age [Leung A.K.C. et al. Dermatology: How to manage acne vulgaris // Drugs in Context. 2020. N. 10.]. Moreover, the prevalence of acne has been growing each year: from 2006 to 2016, an increase of 5.1% has been observed [Hay S.I. et al. Global, regional, and national disability-adjusted life-years (DALYs) for 333 diseases and injuries and healthy life expectancy (HALE) for 195 countries and territories, 1990-2016: A systematic analysis for the Global Burden of Disease Study 2016 // The Lancet. 2017. No. 10100 (390)].

In addition to the discomfort caused by the clinical symptoms of acne, patients may experience other negative effects. A study observed a significantly higher unemployment rate among patients with acne compared to the control group. Moreover, it was found that acne negatively affects social life, self-esteem, and body image, and is often associated with psychological disorders, including depression and anxiety. Specifically, 30.8% of Chinese residents with acne reported that the condition had a negative impact on their quality of life [Shen Y. et al. Prevalence of acne vulgaris in Chinese adolescents and adults: A community-based study of 17,345 subjects in six cities // Acta Dermato-Venereologica. 2012. No. 1 (92)]. According to one study, the cost of treating acne in Germany amounts to 400 million euros annually, which burdens the state budget [Radtke M. A., Schäfer I., Augustin M. [Pharmacoeconomy in acneevaluation of benefit and economics]. // Journal der Deutschen Dermatologischen Gesellschaft = Journal of the German Society of Dermatology: JDDG. 2010. No. SUPPL. 1 (8 Suppl 1)]. Thus, acne is a serious issue among the population, affecting not only physical health but also financial, social, and psychological aspects of life.

The typical manifestations of acne include comedones, small papules, closed comedones, and open comedones. Comedones represent an enlargement of the follicular opening with a keratinized plug [Latter G. et al. Targeted topical delivery of retinoids in the management of acne vulgaris: Current formulations and novel delivery systems // Pharmaceutics. 2019. [V. 11. No. 10]. Free fatty acids, entering the skin as a result of follicular rupture, contribute to the inflammatory response. The disease is most commonly presented on the face, but it can also spread to the neck, torso, and arms. Acne is an inflammatory disease involving *Cutibacterium acnes,* leading to subsequent scarring.

Several factors are known to influence the development of acne. The main causes include excessive sebum production, follicular blockage, diet, stress, bacteria, lifestyle, and, of course, hormonal stress. Human skin has pores connected to sebaceous glands. Sebum maintains skin elasticity and protects against bacterial and fungal infections, acting as a barrier. With excess sebum, dead skin cells and dirt become trapped in the pores, leading to blockage, an inflammatory response, and the formation of acne [Bhadra P., Deb A. A Literature Review On acne Due to Hormonal Changes and Lifestyle A Literature Review Onacne Due to Hormonal Changes and Lifestyle // Indian Journal of Natural Sciences. 2020. No. 59 (10)].

Androgens and androgen receptors are the primary regulators of sebaceous glands. During puberty, sebaceous glands enlarge, hormones become more active, and stimulate the production of more sebum. For instance, in men, sebum production increases fivefold compared to women [Bhadra P., Deb A. A Literature Review On acne Due to Hormonal Changes and Lifestyle A Literature Review Onacne Due to Hormonal Changes and Lifestyle // Indian Journal of Natural Sciences. 2020. No. 59 (10).].

It has long been believed that androgens contribute to the development of certain skin pathologies. Although the skin does not play a decisive role in androgen secretion, sebocytes, sweat glands, and dermal papilla cells have the enzymatic capability to convert dehydroepiandrosterone sulfate and androstenedione into more potent androgens - testosterone and 5α-dihydrotestosterone. Dihydrotestosterone is the most active form, involved in many androgen-related skin diseases, such as acne [Sansone G., Reisner R. M. Differential rates of conversion of testosterone to dihydrotestosterone in acne and in normal human skin--a possible pathogenic factor in acne. // The Journal of investigative dermatology. 1971. No. 5 (56)], hirsutism, and androgenetic alopecia. Through genetic and epigenetic mechanisms, dihydrotestosterone binds to the receptor and stimulates the transcription of 5α-reductase genes, the function of sebaceous glands, sebocyte proliferation, and inflammation [Marks D.H. et al. Topical Antiandrogen Therapies for Androgenetic Alopecia and Acne Vulgaris // American Journal of Clinical Dermatology. 2020. [V. 21. No. 2]. Pathologically increased 5α-reductase activity, altered quality of sebum lipids, in addition to follicular hyperkeratosis, inflammation, and *Cutibacterium acnes,* contribute to the pathogenesis of acne across all populations and both sexes [Dréno B. et al. Cutibacterium acnes (Propionibacterium acnes) and acne vulgaris: a brief look at the latest updates // Journal of the European Academy of Dermatology and Venereology. 2018. V. 32]. In the skin of patients with acne, dihydrotestosterone levels are 20 times higher than in normal facial skin [Sansone G., Reisner R. M., Differential rates of conversion of testosterone to dihydrotestosterone in acne and in normal human skin--a possible pathogenic factor in acne. // The Journal of investigative dermatology. 1971. No. 5 (56)]. This fact underlies the involvement of 5α-reductase and hormonal imbalance in the key pathogenic pathways of acne.

The substrates for 5α-reductase include progesterone, corticosterone, deoxycorticosterone, cortisol, and aldosterone. Cortisol, which is actively produced under stress, is easily converted into 5α-dihydrocortisol by 5α-reductase [Escamilla-Cruz M. et al. Use of 5-Alpha Reductase Inhibitors in Dermatology: A Narrative Review // Dermatology and Therapy. 2023. [V. 13. No. 8]. There are two types of 5α-reductase: 5α-reductase type I is predominantly localized in the skin, including sebaceous glands and hair follicles, while type II is localized in the inner root sheath of hair follicles on the scalp, beard, and chest, as well as in the genitalia and prostate. The enzyme of the first type is involved in the pathogenesis of skin diseases, including acne, while type II is involved in the development of alopecia [Andrzej Slominski et al. Steroidogenesis in the skin: Implications for local immune functions // The Journal of Steroid Biochemistry and Molecular Biology, Volume 137,2013]. It has been shown that facial sebaceous glands convert testosterone into dihydrotestosterone solely through 5α-reductase type I [Diane Thiboutot et al. Activity of the Type 1 5α-Reductase Exhibits Regional Differences in Isolated Sebaceous Glands and Whole Skin // Journal of Investigative Dermatology, Volume 105, Issue 2, 1995]. The activity of 5α-reductase type I has been found to be higher in patients with acne [Baulieu EE et al. Dehydroepiandrosterone (DHEA), DHEA sulfate, and aging: Contribution of the DHEage study to a sociobiomedical issue // Proceedings of the National Academy of Sciences of the United States of America. 2000. No. 8 (97).]. In patients with acne, 5α-reductase type I was predominantly localized in the walls of sebaceous glands [Thiboutot D, Bayne E, Thome J, et al. Immunolocalization of 5α-Reductase Isozymes in Acne Lesions and Normal Skin. Arch Dermatol. 2000;136(9):1125-1129].

Increased activity of 5α-reductase type I and hormonal imbalance in the body contributes to the occurrence and development of acne in both men and women. It has been shown that the onset of acne in prepubescent girls and sebum production in both sexes correlate with levels of dehydroepiandrosterone sulfate in the blood serum. Higher serum androgen levels correlate with severe nodular acne in both men and women, and these levels are often within the normal range in mild to moderate acne [Khondker L., Khan S. I. Acne vulgaris related to androgens - a review. // Mymensingh medical journal : MMJ. 2014. [V. 23. No. 1].

Additionally, studies indicate that diet and the consumption of certain foods (skim milk, carbohydrates, bread, chips, chocolate) also play an important role in the development of acne [Dall'Oglio F. et al. Diet and acne: review of the evidence from 2009 to 2020 // International Journal of Dermatology. 2021. [V. 60. No. 6]. *Propionibacterium acnes* is the main causative agent of acne in the presence of hormonal stress. This bacterium is considered part of the normal skin flora but can cause an inflammatory response and promote excessive follicular hyperkeratinization [Das S., Reynolds R. V. Recent Advances in Acne Pathogenesis: Implications for Therapy // American Journal of Clinical Dermatology. 2014. No. 6 (15)]. In the absence of hormonal stress caused by testosterone derivatives, acne does not often develop. The use of comedogenic cosmetic products, such as facial skin care products and nourishing hair products, can cause the formation of comedones and an active inflammatory response.

The prevalence of acne among the population has led to the development of a large number of medicinal and hygienic topical agents. A separate group includes topical antibacterial agents, particularly clindamycin, erythromycin, minocycline, sodium sulfacetamide, and sulfur. These agents are active against *C. acnes* and, consequently, act on the skin's surface, reducing the stimulation of inflammatory lesions. Topical antibiotics, such as clindamycin, erythromycin, and minocycline, show clinical effects within 12 weeks of continuous use. During this time, resistance rates of C. *acnes* to the drug can significantly increase, rendering the therapy ineffective [Dessinioti C, Katsambas A. Antibiotics and Antimicrobial Resistance in Acne: Epidemiological Trends and Clinical Practice Considerations. Yale J Biol Med. 2022 Dec 22;95(4):429-443.]. There are reports of high resistance rates of *S. aureus* to erythromycin and clindamycin in patients with acne [Steward CD et al. Testing for Induction of Clindamycin Resistance in Erythromycin-Resistant Isolates of Staphylococcus aureus. // Clin Microbiol 43. 2005]. Topical antibiotics cause dryness and severe skin irritation [Mohsin N et al. Acne treatment review and future perspectives // Dermatologic Therapy. 2022. N. 35. No. 9]. They are used only in the treatment of moderate to severe acne and are prescribed by a dermatologist. Monotherapy with agents from this group is not recommended due to the high risk of resistance development [Zaenglein AL et al. Guidelines of care for the management of acne vulgaris. J Am Acad Dermatol. 2016;74(5):945-973]. This group of drugs does not normalize hormonal balance and the activity of 5α-reductase type I in skin cells, so the clinically significant effect is delayed and observed only in combination therapy with anti-inflammatory drugs or with 5α-reductase inhibitors.

Antibacterial agents also include azelaic acid, which has shown significant antibacterial activity against *C. acnes* [Graupe K et al. Efficacy and safety of topical azelaic acid (20 percent cream): an overview of results from European clinical trials and experimental reports. // Cutis; cutaneous medicine for the practitioner. 1996. V. 57. No. 1 Suppl.]. Azelaic acid is a natural dicarboxylic acid that also possesses anti-inflammatory, antioxidant, and exfoliating properties [Implications of azelaic acid's multiple mechanisms of action: Therapeutic versatility // Journal of the American Academy of Dermatology. 2008. No. 2 (58).]. It has been previously reported that azelaic acid is a potent inhibitor of 5α-reductase: partial inhibition of the enzyme was observed at concentrations as low as 0.2 mmol/L, and complete inhibition was achieved at 3 mmol/L [D. STAMATIADIS et al. Inhibition of 5α-reductase activity in human skin by zinc and azelaic acid //British Journal of Dermatology, 1988, Volume 119, Issue 5, 627-632]. However, a disadvantage of azelaic acid is its limited penetration through the stratum corneum, which requires the use of high concentrations (around 20%) to ensure the drug's efficacy, thereby increasing the frequency of side effects such as local skin irritation [Sara Al-Marabeh et al. A prodrug approach to enhance azelaic acid percutaneous availability, Pharmaceutical Development and Technology, 2017, 22:4, 578-586]. Additionally, azelaic acid has low water solubility and low thermal stability, complicating its use in cosmetic formulations [Silvia Quaresma et al. Novel Antibacterial Azelaic Acid BioMOFs//Cryst. Growth Des. 2020, 20, 1, 370-382].

Salicylic acid is widely used in the treatment of mild acne. This keratolytic agent dissolves the intercellular matrix that holds epithelial cells together. It also has minor anti-inflammatory, fungistatic, and bacteriostatic effects [Krautheim A., Gollnick H. Transdermal Penetration of Topical Drugs Used in the Treatment of Acne // Clinical Pharmacokinetics. 2003. V. 42. No. 14]. The substance is effectively absorbed through the skin and is non-toxic at low concentrations. However, at concentrations above 20%, salicylic acid can cause local skin peeling and hypersensitivity [Tasleem Arif Salicylic acid as a peeling agent: a comprehensive review, Clinical, Cosmetic and Investigational Dermatology, 8:, 455-461. 2015]. Moreover, the use of high concentrations of salicylic acid in cosmetic products may lead to dizziness, lethargy, and breathing problems [Akhavan A., Bershad S. Topical acne drugs: Review of clinical properties, system exposure, and safety // American Journal of Clinical Dermatology. 2003. V. 4. No. 7.]. Salicylic acid does not normalize hormonal balance or the activity of 5α-reductase type I in skin cells, so its clinical effect is delayed and observed only in cases of acne caused by bacteria.

Sulfur is considered a mild keratolytic and bacteriostatic agent. In keratinocytes, sulfur is reduced to hydrogen sulfide by an unknown mechanism. It is believed that hydrogen sulfide, produced from sulfur, breaks down keratin and exhibits activity against *C. acnes.* The use of sulfur is associated with the production of hydrogen sulfides, which have an unpleasant odor, and with skin dryness [Lin A. N., Reimer R. J., Carter D. M. Sulfur revisited // Journal of the American Academy of Dermatology. 1988. No. 3 (18).]. The incorporation of sulfur into pharmaceutical and cosmetic formulations is complicated by its insolubility in water and low bioavailability - it hardly penetrates the upper layers of the skin [Victoria Pavlovna Erdakova Results of the complex use of nutraceuticals and cosmeceuticals in the prevention of acne // Equipment and technology of food production. 2010. No. 1 (16).].

The class of retinoids includes well-known agents such as tretinoin, adapalene, and tazarotene, which are used for the treatment of acne and other common skin conditions. Several mechanisms of action likely contribute to the usefulness of topical retinoid agents as acne therapy. These include the modulation of keratinocyte proliferation and the inhibition of inflammation. Topical retinoids suppress keratinocyte proliferation, thereby reducing follicular blockage and preventing the formation of microcomedones [Habeshian K. A., Cohen B. A. Current issues in the treatment of acne vulgaris // Pediatrics. 2020. No. 2 (145)]. Additionally, these agents have anti-inflammatory properties. They also help prevent and reduce the appearance of scars and depigmentation [Thiboutot D. M. et al. Practical management of acne for clinicians: An international consensus from the Global Alliance to Improve Outcomes in Acne // Journal of the American Academy of Dermatology. 2018. No. 2 (78).]. Consumers of these products and patients report local skin dryness, peeling, erythema, and, in some cases, burning and irritation [Layton A. M., Ravenscroft J. Adolescent acne vulgaris: current and emerging treatments // The Lancet Child and Adolescent Health. 2023. V. 7. No. 2]. Some topical retinoids are associated with an increased risk of photosensitivity; their daytime use may increase the risk of sunburn [Cunliffe WJ, Caputo R, Dreno B et al. Clinical efficacy and safety comparison of adapalene gel and tretinoin gel in the treatment of acne vulgaris: Europe and U.S. multicenter trials. J Am Acad Dermatol. 1997; 36: S126-S134].

Benzoyl peroxide is a potent topical antimicrobial agent. It can inhibit the breakdown of triglycerides in skin sebum and reduce inflammation associated with acne. Additionally, benzoyl peroxide has a moderate keratolytic effect [Zaenglein A. L. Acne Vulgaris // New England Journal of Medicine. 2018. No. 14 (379). P. 1343-1352.] After benzoyl peroxide is broken down into benzoic acid and hydrogen peroxide, this agent decreases the population of *C*. *acnes.* Thus, the use of benzoyl peroxide does not contribute to the development of microbial resistance, unlike topical antibiotics [Zaenglein AL. Acne Vulgaris. N Engl J Med. 2018 Oct 4;379(14): 1343-1352]. However, it has been shown that high concentrations of this drug, starting at 5%, can cause irritant dermatitis [Sagransky M, Yentzer BA, Feldman SR. Benzoyl peroxide: a review of its current use in the treatment of acne vulgaris. Expert Opin Pharmacother. 2009 Oct;10(15):2555-62]. Benzoyl peroxide can increase transepidermal water loss by altering the skin's barrier function [Thiboutot D, Del Rosso JQ. Acne Vulgaris and the Epidermal Barrier: Is Acne Vulgaris Associated with Inherent Epidermal Abnormalities that Cause Impairment of Barrier Functions? Do Any Topical Acne Therapies Alter the Structural and/or Functional Integrity of the Epidermal Barrier? J Clin Aesthet Dermatol. 2013 Feb;6(2): 18-24.]. It has been shown that the solubility of benzoyl peroxide is inversely related to its stability, and that the stability of the substance is significantly influenced by the solvent used in the product formula and the process temperature [Chellquist, EM, Gorman, WG Benzoyl Peroxide Solubility and Stability in Hydric Solvents. Pharm Res 9, 1341-1346 (1992)]. This creates challenges in incorporating benzoyl peroxide into formulations.

All of the aforementioned therapeutic components, despite their use in acne therapy, have a number of drawbacks. They cause significant side effects such as skin dryness and irritation and address only the visible symptoms of the disease, without attempting to target its root cause - the increased expression and activity of 5α-reductase type I during hormonal stress in humans.

An attempt to address the root cause of the disease, specifically the increased sebum secretion due to elevated androgen levels in the skin, involves the use of drugs aimed at inhibiting 5α-reductase. One such drug is dutasteride, which is capable of inhibiting both type I and type II 5α-reductases and is recommended for oral administration. To date, there are very few studies evaluating the side effects associated with the use of this substance. Most of the conducted experiments use high doses or have small sample sizes. Currently, there is insufficient research on the long-term side effects of these drugs, despite the fact that patients will be taking them indefinitely. However, the U.S. National Institutes of Health has mandated that depression be listed as a potential side effect on the drug's packaging [Hirshburg J. M. [et al.]. Adverse effects and safety of 5-alpha reductase inhibitors (finasteride, dutasteride): A systematic review // Journal of Clinical and Aesthetic Dermatology. 2016. V. 9. No. 7]. Patients have also reported complaints of headaches, gastrointestinal discomfort, and decreased libido [Roehrborn CG, Boyle P, Nickel JC, et al. Efficacy and safety of a dual inhibitor of 5-alpha-reductase types 1 and 2 (dutasteride) in men with benign prostatic hyperplasia. Urology. 2002;60:434-441.]. Additionally, by inhibiting both types of 5α-reductase, dutasteride inhibits the production of dihydrotestosterone (DHT) throughout the body. Although this hormone is involved in the pathogenesis of acne, it also has a number of important physiological functions, such as improving blood flow rate and regulating prostate volume. The absence of DHT production in the body can lead to disruptions in sexual development [Roehrborn CG et al. Efficacy and safety of a dual inhibitor of 5-alpha-reductase types 1 and 2 (dutasteride) in men with benign prostatic hyperplasia. Urology. 2002 Sep;60(3):434-41.].

Thus, the search for effective components and their combinations as a composition for eliminating acne caused by hormonal imbalance and increased activity of 5α-reductase remains a relevant task for researchers. One promising and innovative approach is the search for components capable of reducing 5α-reductase activity to baseline levels in the skin, thereby preventing the development of acne under hormonal stress conditions characterized by elevated levels of cortisol, dihydrotestosterone, and testosterone. In particular, such solutions include shikimic acid of plant origin and the extract of ume (Japanese apricot) fruit. The authors propose using a composition of these two components in specific mass ratios to mitigate the consequences of hormonal imbalance and the pathogenesis of acne by reducing the activity and quantity of 5α-reductase type I in sebocytes of the skin.

*Shikimic acid* (CAS 138-59-0) is an organic monobasic trihydroxy monocarboxylic acid, extracted, among other sources, from the fruits and seeds of star anise. Despite the fact that shikimic acid was first isolated in the 19th century, its application in cosmetology began only recently, so there are not many studies dedicated to its dermatological use. Shikimic acid, at low concentrations, can exert a deodorizing effect by inhibiting the activity of the lipase enzyme and blocking the production of fatty acids [Garbossa W. A. C., Mercurio D. G., Campos P. M. B. G. M. Shikimic acid: A potential active principle for skin exfoliation // Surgical and Cosmetic Dermatology. 2014. No. 3 (6)]. Additionally, it has an exfoliating effect [Chen Y. H. [et al.]. Skin whitening capability of shikimic acid pathway compounds // European Review for Medical and Pharmacological Sciences. 2016. No. 6 (20).]. It has been shown that shikimic acid does not affect the skin barrier like some exfoliating agents, and its exfoliating mechanism does not have any adverse effects on skin hydration. Shikimic acid also has the ability to restore the epidermal hydrolipid barrier, reducing skin dryness and sensitivity [Kim T.-H. et al. The effects of topical alpha-hydroxyacids on the normal skin barrier of hairless mice // British Journal of Dermatology. 2001. No. 2 (144). P. 267-273.]. It exhibits antibacterial properties, being effective against gram-positive bacteria [Bai J. et al. Antibacterial activity of shikimic acid from pine needles of Cedrus deodara against Staphylococcus aureus through damage to cell membrane // International Journal of Molecular Sciences. 2015. No. 11 (16)]. Thus, shikimic acid has a wide range of beneficial effects for human skin, including antibacterial, exfoliating, and moisturizing effects, without causing irritation, redness, or dryness [Batory M., Rotsztejn H., Shikimic acid in the light of current knowledge // Journal of Cosmetic Dermatology. 2022. No. 2 (21). P. 501-505].

However, there are no data on the use of this substance for the treatment of acne or its effect on 5α-reductase activity during hormonal skin stress. The absence of data presents a scientific interest in studying the effects of shikimic acid on hormonal skin stress induced by high levels of steroid hormones and increased 5α-reductase activity. The widespread adoption of shikimic acid is hindered by its instability in formulations. Many components derived from natural sources with pharmaceutical potential exhibit low stability due to oxidation or intermolecular reactions [Taofiq O. et al. Hydroxycinnamic acids and their derivatives: Cosmeceutical significance, challenges and future perspectives, a review, 2017]. Due to its acidic properties (pKa = 4.15), shikimic acid can lower the pH of a product, leading to its destabilization [Batory M., Rotsztejn H. Shikimic acid in the light of current knowledge, 2022]. The literature describes the low stability of compounds related to shikimic acid, such as polyphenols and hydroxycinnamic acids. One method of stabilizing these compounds, including phenolic acids such as shikimic acid, is encapsulation [Bartosz T., Irene T. Polyphenols encapsulation-application of innovation technologies to improve stability of natural products, 2016; Taofiq O. et al., 2017; Taofiq O. et al. Phenolic acids, cinnamic acid, and ergosterol as cosmeceutical ingredients: Stabilization by microencapsulation to ensure sustained bioactivity, 2019]. Shikimic acid is poorly soluble in non-polar and hydrophobic compounds, such as those used in acne patches, but it is highly soluble in water (180 g/L) [Saptarshi Ghosh et al Production of shikimic acid / Biotechnology Advances, Volume 30, Issue 6, 2012].

*Prunus Mume Fruit Extract* is a multicomponent plant extract derived from the fruits of the Japanese plum, one of the oldest medicinal plants widely used in the East. To date, phytochemical studies have identified numerous chemical components of the plant, mainly phenols, flavonoids, and organic acids [Gong X. P. [et al.]. Comprehensive Review of Phytochemical Constituents, Pharmacological Properties, and Clinical Applications of Prunus mume // Frontiers in Pharmacology. 2021. V. 12]. Several studies have shown that *Prunus Mume Fruit Extract* exhibits a broad spectrum of antibacterial activity due to its phenolic compounds [Seneviratne CJ et al. Prunus mume extract exhibits antimicrobial activity against pathogenic oral bacteria // International Journal of Paediatric Dentistry. 2011. No. 4 (21)]. Various studies have also found that *P. mume* has anti-inflammatory activity. Triterpenoid compounds isolated from ume fruits, such as oleanolic acid, inhibited the release of pro-inflammatory mediators from lipopolysaccharide-stimulated cells [Morimoto-Yamashita Y. et al. A natural therapeutic approach for the treatment of periodontitis by MK615 // Medical Hypotheses. 2015. No. 5 (85)]. The ethanol extract of Japanese plum fruits attenuated H2O2-induced oxidative stress and apoptosis in cells. An acylated quinic acid isolated from ume fruit extract inhibited melanogenesis and showed no cytotoxicity, indicating a potential skin-whitening effect [Lee SW et al. Effects of Prunus mume Siebold & Zucc. in the pacemaking activity of interstitial cells of Cajal in murine small intestine // Experimental and Therapeutic Medicine. 2017. No. 1 (13)]. Thus, Prunus Mume Fruit Extract exhibits anti-inflammatory, skin-whitening, and antioxidant effects. There is a study focused on testing Prunus Mume Fruit Extract on the scalp skin to inhibit 5α-reductase [Lee SH et al. Extracts for the Hair Growth Stimulation using In vivo and In vitro Test Models // The Journal of Korean Medicine Ophthalmology and Otolaryngology and Dermatology. 2002]. In this study, the substance showed effective potential in inhibiting the activity of the target enzyme. However, the study was entirely dedicated to the stimulation of hair growth, so all experiments were conducted on scalp skin models where the 5α-reductase family is represented by type II 5α-reductase. There are no research results on the effect of Japanese plum fruit extract on the activity of type I 5α-reductase, so the data cannot be extrapolated from the aforementioned study.

The use of Prunus Mume fruit extract in cosmetics can be challenging due to its high content of flavonoids, such as caffeic acid, p-coumaric acid, ferulic acid, squalene, catechin, epicatechin, citric acid, rutin, asparagine, and others [Xingna Wang et al. Antibiotic activities of extracts from Prunus mume fruit against food-borne bacterial pathogens and its active components // Industrial Crops and Products, Volume 133, 2019]. It has been reported that some polyphenols, such as rutin, precipitate in aqueous solutions of products [Löf D, Schillén K, Nilsson L. Flavonoids: precipitation kinetics and interaction with surfactant micelles. J Food Sci. 2011 Apr;76(3):N35-9.]. Ultraviolet exposure can also lead to the degradation of compounds such as catechin [Volf, I., Ignat, I., Neamtu, M. et al. Thermal stability, antioxidant activity, and photooxidation of natural polyphenols. Chem. Pap. 68, 121-129 (2014)]. In one study, plant extracts rich in polyphenols showed an unpredictable ability to alter the rheological properties of hydrocolloid films depending on the substances used: they could increase or decrease solution viscosity, change thermal stability, and affect fluidity [Silva-Weiss A. et al. Polyphenol-rich extract from murta leaves on rheological properties of film-forming solutions based on different hydrocolloid blends // Journal of Food Engineering, Volume 140, 2014].

### SUMMARY OF THE INVENTION

In a first aspect, the invention relates to a biocomplex for body skin care, specifically for managing hormonal skin stress and the epigenetic modulation of 5α-reductase type I in skin cells, particularly epidermal cells, sebocytes, and hair follicle cells. The invention relates to a biocomplex consisting of components A and B, where:
(A) Shikimic acid or its salts, shikimates, of plant origin;
(B) Extract of Japanese apricot *Prunus Mume,*
where the mass ratio of the components, in terms of active substances, is (0.10-1.0):(0.001-1.0) wt. % respectively.

The composition is characterized by the fact that shikimic acid or its salts, shikimates, represent a substance or commercially available shikimic acid with CAS registration number 138-59-0 . Shikimic acid may be Shikimic acid, a plant extract of Illicium verum (Anise) Fruit Extract standardized for shikimic acid content, or other commercially available raw materials.

The composition is characterized by the fact that the plant extract from *Prunus Mume* represents a substance or a commercially available plant extract from the fruits, seeds, leaves, or whole parts of *Prunus Mume.* The plant extract from *Prunus Mume* leaves *may be Prunus Mume Fruit Extract,* such as Prunus Mume Fruit Ext.(H) Danjoungbio or Ume extract (B)-BG Nikkol Japan, or other commercially available raw materials.

According to the invention, *Prunus Mume Fruit Extract* may be a dry extract, aqueous extract, alcoholic extract, aqueous-alcoholic extract, aqueous-glycerin extract, oil extract, or CO₂ extract, preferably an aqueous-glycerin or dry extract.

The composition according to the invention may contain the following, wt. %:

| | |
|---|---|
| Shikimic acid or its salts, shikimates | 0.10-1.00%, |
| *Prunus Mume* fruit extract | 0.001-1.00%. |

In a second aspect, the invention relates to combined skin care products, particularly acne patches or transdermal patches, containing 0.1-2.00% of the composition according to the invention.

In a third aspect, the invention relates to skin care products for acne-prone facial skin, particularly creams, lotions, serums, or tonics, containing 0.1-2.00% of the composition according to the invention.

In a fourth aspect, the invention relates to cleansing products for acne-prone facial skin, particularly cleansing gels, foams, cleansing balms, masks, or powders, containing 0.1-2.00% of the composition according to the invention.

In a fifth aspect, the invention relates to hair care products, particularly shampoos, lotions, or serums, containing 0.1-2.00% of the composition according to the invention.

In a sixth aspect, the invention relates to the use of the composition according to the invention for managing hormonal skin stress and the epigenetic modulation of 5α-reductase type I in skin cells, particularly epidermal cells, sebocytes, and hair follicle cells, as part of various perfumery and cosmetic products.

Optionally the composition may contain exosomes and plant extract. The plant extract may be represented by clover, especially Chinese bush-clover, particularly Lespedeza cuneata extract.

### DETAILED DESCRIPTION OF THE INVENTION

The claimed invention relates to a biocomplex for body skin care, specifically for managing hormonal skin stress and the epigenetic modulation of 5α-reductase type I in skin cells, particularly in epidermal cells, sebocytes, and hair follicle cells. The biocomplex consists of shikimic acid or its salts, shikimates, of plant origin, and *Prunus Mume* fruit extract, taken in a mass ratio of (0.10-1.0):(0.001-1.00) wt. % respectively.

Shikimic acid or its salts, shikimates, of plant origin, particularly shikimic acid (INCI: *Shikimic acid*), is a commercially available cosmetic ingredient well-known to specialists in the field.

Prunus Mume fruit extract (INCI: *Prunus Mume Fruit Extract*) is also a commercially available cosmetic ingredient well-known to specialists in the field.

In the composition of the invention, *Prunus Mume* fruit extract may be a dry extract, an aqueous extract obtained by extraction in hot or cold water, an alcoholic extract, an aqueous-alcoholic extract, an aqueous-glycerin extract obtained by extraction in alcohol, alcohol diluted with water from 96% alcohol, or glycerin, an oil extract obtained by extraction in cold oil (maceration) or hot oil (infusion), or a CO2 extract obtained from plant raw materials by extraction with liquefied carbon dioxide under high pressure and low temperature, preferably a dry or aqueous-glycerin extract. The production of dry extracts can be carried out according to two schemes. In the first case, the process consists of four stages: 1) obtaining the extract, 2) purifying the extract, 3) concentrating the extract, and 4) drying the concentrated extract. In the second case, the production process does not include the concentration stage. To standardize dry extracts by active substance, fillers such as lactose, glucose, potato dextrin, maltodextrin, etc., are used. (https://ztl.nuph.edu.ua/medication/chapter05.html). These extracts are well-known to specialists in the field and are commercially available from various suppliers.

In the composition of the invention, *Prunus Mume* fruit extract may be an aqueous extract of *Prunus Mume* fruit, with or without the addition of preservatives such as sodium benzoate and potassium sorbate, glycerin, 1,2-hexanediol, other solvents, and pH regulator citric acid.

The composition according to the invention may contain the following, wt. %:

| | |
|---|---|
| Shikimic acid or its salts, shikimates | 0.10-1.00%, |
| *Prunus Mume* fruit extract | 0.001-1.00%. |

In the composition of the invention, acceptable auxiliary substances may be selected from the following categories of components, but not necessarily depending on the formula of the finished product with the composition according to the invention.

Anionic Surfactants:
1. Salts of higher carboxylic acids with the general formula: R1-CO2X1, where R1 is an alkyl and/or alkenyl group with a hydrocarbon chain length of 5 to 21 carbon atoms, and X1 is a cation of an alkali and/or alkaline earth metal, ammonium, alkylammonium, alkanolammonium, glucoammonium, or a basic amino acid;
2. Salt of a higher fatty acid amide and methylglycine with the general formula R4-C(O)-N(-CH3)-CH2-CO2X4, where R4 is an alkyl and/or alkenyl group with a hydrocarbon chain length of 5 to 21 carbon atoms, and X4 is a cation of an alkali and/or alkaline earth metal, ammonium, alkylammonium, alkanolammonium, glucoammonium;
3. Alkyl polyethylene glycol carboxylate with the general formula: R5-O(-CH2-CH2-O-)n2CH2-CO2X5, where n2 can range from 1 to 15, indicating the number of polyethylene glycol groups, R5 is an alkyl and/or alkenyl group with a hydrocarbon chain length of 6 to 22 carbon atoms, and X5 is a cation of an alkali and/or alkaline earth metal, ammonium, alkylammonium, alkanolammonium, glucoammonium;
4. Alkyl sulfate with the general formula R3-OSO3X3, where R3 is an alkyl and/or alkenyl group with a hydrocarbon chain length of 6 to 22 carbon atoms, and X3 is a cation of an alkali and/or alkaline earth metal, ammonium, alkylammonium, alkanolammonium, glucoammonium;
5. Alkyl polyethylene glycol sulfate with the general formula: R2-O(-CH2-CH2-O)n1SO3X2, where n1 can range from 1 to 10, indicating the number of polyethylene glycol groups, R2 is an alkyl and/or alkenyl group with a hydrocarbon chain length of 6 to 22 carbon atoms, and X2 is a cation of an alkali and/or alkaline earth metal, ammonium, alkylammonium, alkanolammonium, glucoammonium;
6. Disubstituted salt of 2-sulfo carboxylic acid with the general formula: R6-CH(-SO3X6)-CO2X6, where R6 is an alkyl and/or alkenyl group with a hydrocarbon chain length of 4 to 20 carbon atoms, and X6 is a cation of an alkali and/or alkaline earth metal, ammonium, alkylammonium, alkanolammonium, glucoammonium;
7. Mono- or disubstituted salt of a higher carboxylic acid amide and glutamic acid with the general formula: R7-C(O)-NH-CH(-CH2-CH2-CO2X7)-CO2X7, where R7 is an alkyl and/or alkenyl group with a hydrocarbon chain length of 5 to 21 carbon atoms, and X7 is a cation of an alkali and/or alkaline earth metal, ammonium, alkylammonium, alkanolammonium, glucoammonium, or hydrogen;
8. Salt of a higher fatty acid amide and glycine with the general formula: R8-C(O)-NH-CH2-CO2X8, where R8 is an alkyl and/or alkenyl group with a hydrocarbon chain length of 5 to 21 carbon atoms, and X8 is a cation of an alkali and/or alkaline earth metal, ammonium, alkylammonium, alkanolammonium, glucoammonium;
9. Salt of a higher fatty acid amide and alanine with the general formula: R9-C(O)-NH-CH(-CH3)-CO2X9, where R9 is an alkyl and/or alkenyl group with a hydrocarbon chain length of 5 to 21 carbon atoms, and X9 is a cation of an alkali and/or alkaline earth metal, ammonium, alkylammonium, alkanolammonium, glucoammonium;
10. Salt of a higher fatty acid amide and 2-aminomethanesulfonic acid with the general formula: R10-C(O)-N(-CH3)-CH2-CH2-SO3X10, where R10 is an alkyl and/or alkenyl group with a hydrocarbon chain length of 5 to 21 carbon atoms, and X10 is a cation of an alkali and/or alkaline earth metal, ammonium, alkylammonium, alkanolammonium, glucoammonium;
11. Alkylpolyglucoside hydroxypropylsulfonate with the general formula: R11-O-[G]p1-O-CH2-CH(-OH)-CH2-SO3X11, where R11 is an alkyl and/or alkenyl group with a hydrocarbon chain length of 6 to 22 carbon atoms, G is a saccharide fragment containing 5 or 6 carbon atoms, p1 can range from 1 to 4, and X11 is a cation of an alkali and/or alkaline earth metal, ammonium, alkylammonium, alkanolammonium, glucoammonium;
12. Alkylpolyglucoside carboxylate with the general formula: R12-O-[G]p2-O-CH2-CO2X12, where R12 is an alkyl and/or alkenyl group with a hydrocarbon chain length of 6 to 22 carbon atoms, G is a saccharide fragment containing 5 or 6 carbon atoms, p2 can range from 1 to 4, and X12 is a cation of an alkali and/or alkaline earth metal, ammonium, alkylammonium, alkanolammonium, glucoammonium;
13. Salt of a higher fatty acid amide and threonine with the general formula: R13-C(O)-NH-CH(-CH(-OH)-CH3)-CO2X13, where R13 is an alkyl and/or alkenyl group with a hydrocarbon chain length of 5 to 21 carbon atoms, and X13 is a cation of an alkali and/or alkaline earth metal, ammonium, alkylammonium, alkanolammonium, glucoammonium;
14. Salt of a higher fatty acid amide and an amino acid obtained by the hydrolysis of plant proteins with the general formula: R14-C(O)-AAX14, where R14 is an alkyl and/or alkenyl group with a hydrocarbon chain length of 5 to 21 carbon atoms, AA is an amino acid or peptide obtained by hydrolysis of plant protein (possible protein sources: apple, soy, wheat, cotton, etc.), and X14 is a cation of an alkali and/or alkaline earth metal, ammonium, alkylammonium, alkanolammonium, glucoammonium.
15. Rhamnolipids, which are glycolipid surfactants of microbial origin. The rhamnose fragments may be present as either mono- (single) or di- (double) units linked by α-1,2-glycosidic bonds. The lipid fragments are connected by ester bonds between β-hydroxyl groups and can be saturated, monounsaturated, or polyunsaturated, with chain lengths ranging from C8 to C16.

Amphoteric Surfactants:
1. Disubstituted salt of acylamphodiacetate with the general formula: R15-C(O)-NH-CH2-CH2-N(-CH2-CO2X15)-CH2-CH2-O-CH2-CO2X15, where R15 is an alkyl and/or alkenyl group with a hydrocarbon chain length of 5 to 21 carbon atoms, and X15 is a cation of an alkali and/or alkaline earth metal, ammonium, alkylammonium, alkanolammonium, glucoammonium;
2. Salt of acylamphoacetate with the general formula: R16-C(O)-NH-CH2-CH2-N(-CH2-CO2X16)-CH2-CH2-OH, where R16 is an alkyl and/or alkenyl group with a long hydrocarbon chain of 5 to 21 carbon atoms, and X16 is a cation of an alkali and/or alkaline earth metal, ammonium, alkylammonium, alkanolammonium, glucoammonium;
3. Salt of alkylamphoacetate with the general formula: R17-C(=N-CH2-CH2-N((-CH2-CH2-OH)-CH2-CO2X17)-), where R17 is an alkyl and/or alkenyl group with a hydrocarbon chain length of 5 to 21 carbon atoms, and X17 is a cation of an alkali and/or alkaline earth metal, ammonium, alkylammonium, alkanolammonium, glucoammonium;
4. Acylamidoalkylbetaine with the general formula: R18-C(O)-NH-R19-N(-CH3)2)-CH2-CO2, where R18 is an alkyl and/or alkenyl group with a hydrocarbon chain length of 5 to 21 carbon atoms, and R19 is an alkyl group with a hydrocarbon chain length of 1 to 4 carbon atoms;
5. Acylamidoalkylhydroxysultaine with the general formula: R20-C(O)-NH-R21-N(-CH3)2-CH2-CH(-OH)-CH2-SO3, where R20 is an alkyl and/or alkenyl group with a hydrocarbon chain length of 5 to 21 carbon atoms, and R21 is an alkyl group with a hydrocarbon chain of 1 to 4 carbon atoms;
6. Acylamidoalkylamine oxide with the general formula: R22-C(O)-NH-R23-N(-CH3)2-O, where R22 is an alkyl and/or alkenyl group with a hydrocarbon chain length of 5 to 21 carbon atoms, and R23 is an alkyl group with a hydrocarbon chain of 1 to 4 carbon atoms;
7. Alkylbetaine with the general formula: R24-N(-CH3)2)-CH2-CO2, where R24 is an alkyl and/or alkenyl group with a hydrocarbon chain length of 5 to 21 carbon atoms;
8. Alkylhydroxysultaine with the general formula: R25-N(-CH3)2-CH2-CH(-OH)-CH2-SO3, where R25 is an alkyl and/or alkenyl group with a hydrocarbon chain length of 6 to 22 carbon atoms;
9. Alkylsultaine with the general formula: R26-N(-CH3)2-CH2-CH2-CH2-SO3, where R26 is an alkyl and/or alkenyl group with a hydrocarbon chain length of 6 to 22 carbon atoms;
10. Alkylamine oxide with the general formula: R27-N(-CH3)2-O, where R27 is an alkyl and/or alkenyl group with a hydrocarbon chain length of 6 to 22 carbon atoms.

Non-Ionic Surfactants:
1. Alkyl polyethylene glycol with the general formula: R29-O(-CH2-CH2-O-)n3H, where n3 can range from 2 to 20, indicating the number of polyethylene glycol groups, and R29 is an alkyl and/or alkenyl group with a hydrocarbon chain length of 6 to 22 carbon atoms;
2. Alkyl polyethylene/polypropylene glycol with the general formula: R30-O(-CH2-CH2-O-)n4(-CH(-CH3)-CH2-O-)n5H, where n4 can range from 2 to 20, indicating the number of polyethylene glycol groups, n5 can range from 2 to 20, indicating the number of polypropylene glycol groups, and R30 is an alkyl and/or alkenyl group with a hydrocarbon chain length of 6 to 22 carbon atoms;
3. Dialkyl polyethylene glycol with the general formula: R31-O(-CH2-CH2-O-)n6R32, where n6 can range from 2 to 20, indicating the number of polyethylene glycol groups, R31 is an alkyl and/or alkenyl group with a hydrocarbon chain length of 6 to 22 carbon atoms, and R32 is an alkyl and/or alkenyl group with a hydrocarbon chain of 1 to 12 carbon atoms;
4. Dialkyl polyethylene/polypropylene glycol with the general formula: R33-O(-CH2-CH2-O-)n7(-CH(-CH3)-CH2-O-)n8-R34, where n7 can range from 2 to 20, indicating the number of polyethylene glycol groups, n8 can range from 2 to 20, indicating the number of polypropylene glycol groups, R33 is an alkyl and/or alkenyl group with a hydrocarbon chain length of 6 to 22 carbon atoms, and R34 is an alkyl and/or alkenyl group with a hydrocarbon chain of 1 to 12 carbon atoms.

Solvents:
1. Organic alcohol with the general formula: R35(-OH)s1, where R35 is an alkyl group with a hydrocarbon chain of 3 to 12 carbon atoms, and S1 can range from 1 to 12, indicating the number of hydroxyl groups positioned arbitrarily in the hydrocarbon radical relative to each other;
2. Alkyl polypropylene glycol with the general formula: H(-CH(-CH3)-CH2-O-)n9R36, where n9 can range from 2 to 10, indicating the number of polypropylene glycol groups, and R36 is an alkyl group with a hydrocarbon chain of 1 to 10 carbon atoms.

pH Regulators:
1. Organic acids with the general formula: R37(-OH)s2(-COOH)m1, where R37 is an alkyl group with a hydrocarbon chain of 1 to 12 carbon atoms, S2 can range from 1 to 12, indicating the number of hydroxyl groups positioned arbitrarily in the hydrocarbon radical relative to each other, and m1 can range from 1 to 4, indicating the number of carboxyl groups positioned arbitrarily in the hydrocarbon radical relative to each other;
2. Solutions of alkali or alkaline earth metal hydroxides, ammonia, primary and tertiary alkylamines, primary and tertiary alkanolamines, primary and tertiary gluconamines, basic amino acids, disodium citrate, trisodium citrate.

Chelating Agents or Complexing Agents:
1. Trisodium salt of methylglycinediacetic acid, tetrasodium salt of glutaminediacetic acid, trisodium salt of ethylenediamine (N,N)-disuccinate;
2. Organic acids, as well as their salts of alkali metals, ammonium, alkylammonium, alkanolammonium, glucoammonium, corresponding to these acids: citric acid, malic acid, tartaric acid, glutaric acid, adipic acid, glucuronic acid, galacturonic acid, galactaric acid, gluconic acid, phytic acid, polylactic acid, polyacrylic acid, polymethacrylic acid, copolymer of acrylic and maleic acids, as well as organic acids with the general formula R38(-OH)s3(-COOH)m2, where R38 is an alkyl group with a hydrocarbon chain of 1 to 12 carbon atoms, S3 can range from 1 to 12, indicating the number of hydroxyl groups positioned arbitrarily in the hydrocarbon radical relative to each other, and m2 can range from 1 to 4, indicating the number of carboxyl groups positioned arbitrarily in the hydrocarbon radical relative to each other.

Solvents: glycerin, propylene glycol, propanediol, 1,2-hexanediol, butylene glycol, pentylene glycol, or others.

Polymer Additives: cellulose gum, polyisobutene, acrylates copolymer, sodium acrylate/sodium acryloyldimethyl taurate copolymer, or others.

Preservatives:
1. Organic acids and their salts of alkali and alkaline earth metals, ammonium, alkylammonium, alkanolammonium, glucoammonium, corresponding to these acids: benzoic acid, sorbic acid, 4-methoxybenzoic acid, salicylic acid, undecylenic acid;
2. Organic alcohols and phenols: phenoxyethanol, benzyl alcohol, caprylyl glycol, ethylhexylglycerin, phenethyl alcohol, 3-methyl-4-isopropylphenol, 2,4-dichlorobenzyl alcohol;
3. Broad-spectrum biocides: benzisothiazolinone, methylisothiazolinone, dodecyldipropylene triamine;
4. Fungicides: sodium pyrithione, climbazole.
5. Plant extracts with preservative function: Lonicera Caprifolium flower extract, Lonicera Japonica (Honeysuckle) Flower Extract, Illicium Verum (Anise) Fruit Extract, Usnea Barbata (Lichen) Extract, Ocimum Basilicum (Basil) Flower/Leaf Extract, Levulinic acid with Hinokitiol, Citrus Paradisi Seed extract with Ascorbic Acid, Citrus reticulata fruit extract, Citrus aurantium amara fruit extract, Citrus aurantium sinensis peel extract, Magnolia Officinalis Bark Extract, individually or in combination with additional components.

Fragrances with or without essential oils of any olfactory direction to enhance consumer properties.

Essential oils in pure form or as mixtures in various ratios: orange, bergamot, lemon, lime, mandarin, grapefruit, neroli, rosewood, yuzu, lemongrass, lavender, sage, thyme, melissa, mint of various botanical species, tea tree, eucalyptus, cedar, sandalwood, black pepper, pink pepper, cinnamon, cardamom, coriander, jasmine, rose, peony, blue chamomile, basil, geranium, copaiba, juniper, as well as any other commercially available essential oils known to specialists in the field.

### EXAMPLES

The examples included in this description are not intended to limit the claimed invention but are provided solely to illustrate and confirm the achievement of the expected technical results. These examples are among many experimental data obtained by the inventors, which confirm the efficacy of the compositions within the scope of the invention.

### Example 1.

The components for inclusion in the composition according to the invention were tested in various perfumery and cosmetic products. A liquid skin care and cleansing product, particularly thin acne patches, was prepared as part of the invention (Table 1).

**Table 1. Composition of thin acne patches with the claimed composition according to the invention**

| **No.** | **Component** | **Content, wt. %** |
|---|---|---|
| 1 | Water | up to 100.00 |
| 2 | Acrylates Copolymer (water mixture 1:1) | 88.10 |
| 3 | Sodium Acrylate/Sodium Acryloyldimethyl Taurate Copolymer | 1.20 |
| 4 | 1,2-Hexanediol | 5.60 |
| 5 | Polyisobutene | 0.80 |
| 6 | Solubilizers (caprylyl/capric glucoside, sorbitan oleate) | 0.20 |
| 7 | Gluconolactone | 1.00 |
| 8 | Shikimic acid | 0.50 |
| 9 | Prunus Mume Fruit Extract | 0.50 |
| 10 | Lespedeza Cuneata Extract | 0.25 |
| 11 | Lactobacillus Ferment Extract | 0.25 |
| 12 | Additives (glycerin, butylene glycol, 1,2-hexanediol) | up to 3.00 |

The prepared thin patch for acne treatment provides sufficient absorbency, adheres tightly to the skin, is hypoallergenic, eliminates early-stage pimples, prevents post-acne formation, and manages hormonal skin stress and the epigenetic modulation of 5α-reductase type I in skin cells (epidermal cells, sebocytes) in cases of acne or isolated pimples.

### Example 2.

The components for inclusion in the composition according to the invention were tested in various perfumery and cosmetic products. A liquid skin care and cleansing product, particularly hydrocolloid absorbing acne patches, was prepared as part of the invention (Table 2).

**Table 2. Composition of hydrocolloid acne patches with the claimed composition according to the invention**

| **No.** | **Component** | **Content, wt. %** |
|---|---|---|
| 1 | Cellulose Gum | 42.00 |
| 2 | Polyisobutene | 57.80 |
| 3 | Shikimic acid | 0.10 |
| 4 | Prunus Mume Fruit Extract | 0.10 |
| 5 | Additives (glycerin, butylene glycol, 1,2-hexanediol) | up to 1.00 |

The prepared hydrocolloid patch for absorbing exudate and drying deep comedones provides sufficient absorbency, adheres tightly to the skin, is hypoallergenic, prevents post-acne formation, and manages hormonal skin stress and the epigenetic modulation of 5α-reductase type I in skin cells (epidermal cells, sebocytes) in cases of acne or isolated pimples.

### Example 3.

An in vitro study was conducted to assess the cytotoxicity of the composition components on dermal sebocytes after the use of the composition components according to the invention.

The test methodology was based on evaluating the cytotoxicity of the components depending on the concentration of the components from the composition, determining the number of surviving cells by the MTT colorimetric method. For the assay, the product was prepared in culture medium with 3% Tween 20 (Sigma) and added to the 96-well plate at a serial dilution in the range of 100.00 to 0.003 mg/mL using a dilution factor of 3.1610. Sebocyte cells were used at a density of 10⁵ cells per well in a 96-well plate. Study conditions: humidified atmosphere with 5% CO₂ and at 37°C. The incubation time was 48 hours. Purified water without the addition of composition components was used as a negative control. The number of surviving cells was recorded using MTT (3-(4,5 dimethylthiazol-2-yl)-2,5-diphenyltetrazolium; Sigma, St. Louis, MO) at a concentration of 5mg/mL (30µL/well), incubated for an additional 4 hours, and absorbance was measured at 570 nm using a Multiskan GO monochromator (Thermo Scientific, Finland). The number of biological replicates was 6. Statistical data processing was performed to calculate the mean value, standard deviation, and trend for each biological replicate. Normality of the data was checked using the Shapiro-Wilk test. Comparison was conducted using a linear mixed-effects model in the case of normality; in the case of non-parametric statistics, the Wilcoxon test was used.

### Results.

The results of the primary cytotoxicity assessment showed that the components of the composition, both individually and in combination, do not cause significant death of skin sebocytes within the claimed concentration range from 0.001% to 2%. The viability of skin sebocytes was maintained throughout the concentration range of the composition components and above the threshold value of 70%, indicating a gentle effect on skin cells involved in the pathogenesis of acne (Table 3).

**Table 3. Assessment of cytotoxicity of composition components on skin sebocytes**

| **Test Sample** | **Composition Components** | **Cell Viability, % when components are added to the culture medium (wt. %)** | | | | | | |
|---|---|---|---|---|---|---|---|---|
| | | **0.001%** | **0.006%** | **0.02%** | **0.06%** | **0.20%** | **0.6%** | **2.0%** |
| No. 1 | Negative control (purified water) | 100.0±2.00 | 100.0±2.00 | 100.0±2.00 | 100.0±2.00 | 100.0±2.00 | 100.0±2.00 | 100.0±2.00 |
| No. 2 | Shikimic acid | 105.0±3.00 | 105.0±3.00 | 105.0±3.00 | 105.0±3.00 | 103.0±3.00 | 104.0±3.00 | 100.0±3.00 |
| No. 3 | *Prunus Mume* Fruit Extract + Shikimic acid (1:1 wt. ratio) | 100.0±3.00 | 100.0±2.00 | 102.0±3.00 | 101.0±3.00 | 99.0±3.00 | 90.0±3.00 | 85.0±3.00 |
| No. 4 | Positive control (salicylic acid) | 90.0±2.00 | 90.0±2.00 | 90.0±2.00 | 85.0±2.00 | 85.0±2.00 | 83.0±2.00 | 81.0±2.00 |

The combination of *Prunus Mume* fruit extract and shikimic acid in a 1:1 mass ratio allowed for the maintenance of high skin sebocyte viability (from 85% to 102% of living cells) throughout the claimed concentration range of 0.001-2.0 wt. % compared to the negative and positive controls, indicating a gentle effect of the composition components on skin cells and a more beneficial effect of the components on the metabolic activity of skin cells. The use of higher concentrations of the composition according to the invention is possible; the components are approved for use in perfumery-cosmetic and pharmaceutical products for skin care and acne prevention. The use of the complex in various dermatological products allows for managing hormonal skin stress and ensuring the epigenetic modulation of 5α-reductase type I in skin cells within the selected concentration range, as shown in Example 4.

### Example 4.

An in vitro study was conducted to determine the amount of 5-alpha reductase type I in skin sebocytes after the use of the composition components according to the invention within the selected concentration range, compared to several positive controls known for the prevention and treatment of acne.

Increased levels of 5α-reductase expression are observed in tissues under hormonal stress [Sánchez P, Torres JM, Gavete P, Ortega E. Effects of swim stress on mRNA and protein levels of steroid 5alpha-reductase isozymes in prefrontal cortex of adult male rats. Neurochem Int., 2008, 52(3):426-31]. It is known that 5α-reductase is one of the main causes of acne development due to the increase in dihydrotestosterone, inflammatory response, and increased sebum secretion. In sebocytes of sebaceous glands, 5α-reductase type I is predominantly localized. It has been shown that facial sebaceous glands convert testosterone into dihydrotestosterone solely through 5α-reductase type I [Diane Thiboutot et al. Activity of the Type 1 5α-Reductase Exhibits Regional Differences in Isolated Sebaceous Glands and Whole Skin // Journal of Investigative Dermatology, Volume 105, Issue 2, 1995]. Increased activity of 5α-reductase type I and hormonal imbalance in the body contributes to the occurrence and development of acne in both men and women.

The test methodology was based on determining the amount of 5α-reductase in skin sebocytes under hormonal stress (in the form of testosterone to simulate physiological conditions in the culture medium) after adding the composition components to the cell culture medium, compared with positive and negative controls. The study was conducted on skin sebocytes after cultivation under standard conditions, using the 3 maximum non-toxic concentrations of the composition components from the previous testing. Additionally, skin sebocytes were stimulated with 10 nM testosterone. Cultivation conditions: humidified atmosphere with 5% CO₂, at 37°C, for 72 hours to simulate inflammatory and hormonal stress. After this period, the cell supernatant was collected, purified, and the amount of 5α-reductase was assessed using ELISA (commercially available kit from ABClonal, USA). Quantification was performed spectrophotometrically at a wavelength of 450 nm using a Multiskan GO monochromator (Thermo Fisher Scientific, Finland).

Purified water was used as a negative control, and salicylic acid and azelaic acid, known for treating acne and related conditions, were used as positive controls for comparison.

To evaluate the data obtained, the ANOVA test was used, which also allowed measuring the variation in results and comparing data between groups. The Bonferroni post-test was then applied, which reinforced and made the result presented in the ANOVA test even more precise. A significance level of 5% was used in both assessments.

### Results.

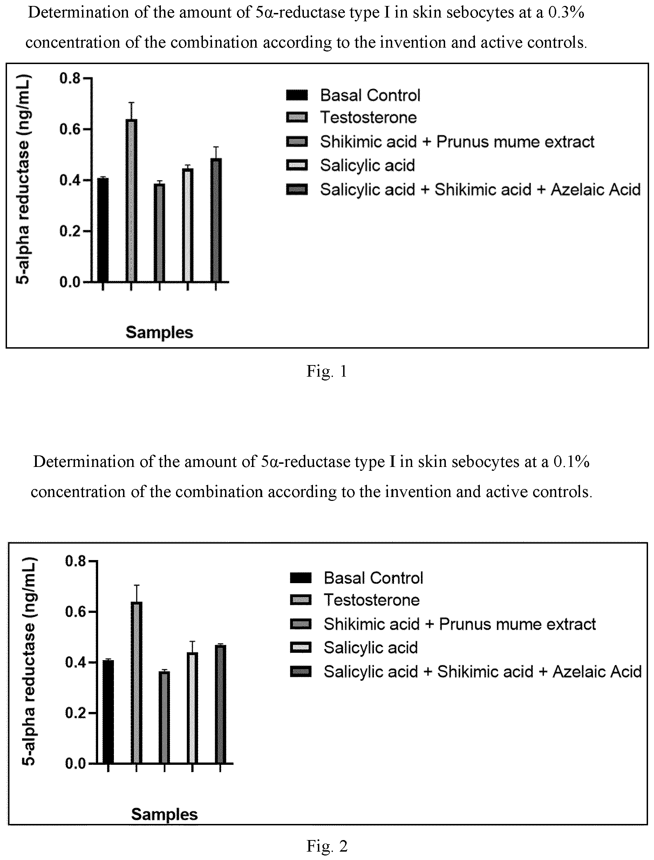

For the study, concentrations of compounds with the highest survival rate of skin cells were selected. The results of determining the amount of 5α-reductase type I in skin sebocytes after adding the composition components to the culture medium revealed a synergistic effect of *Prunus Mume* fruit extract and shikimic acid in a 1:1 mass ratio when used together (Figures 1 and 2). The combination of *Prunus Mume* fruit extract and shikimic acid in a 1:1 mass ratio produced a synergistic effect and reduced the amount of 5α-reductase type I to 110.87% against the background of hormonal inflammatory stress induced by testosterone (10 nM), compared to the baseline level after 48 hours of dermal cell cultivation. This indicates a reduction in the consequences of hormonal stress induced by testosterone and related hormones and modulation of the expression and, consequently, the amount of 5α-reductase type I in response to hormonal stress factors. The combination demonstrates high biological activity in reducing 5α-reductase type I in sebocytes at concentrations from 0.03% to 1% (Table 4). Shikimic acid alone has a more moderate effect on reducing the expression of 5α-reductase type I in skin sebocytes. Thus, for a comparable effect on reducing the amount of 5α-reductase type I, 0.3% shikimic acid is required compared to 0.03% of the combination of shikimic acid and *Prunus Mume* fruit extract in a 1:1 mass ratio, i.e., almost 10 times more for 100% suppression of hormonal stress and its effects on 5α-reductase type I in skin sebocytes (Table 4). *Prunus Mume* fruit extract acts as a synergistic additive, allowing the concentration of shikimic acid to be reduced tenfold while maintaining the high biological activity of the combination. The Prunus Mume fruit extract itself does not have inhibitory activity on 5α-reductase type I according to literature data from public sources (accessed on 04.07.2024). Although Prunus Mume extract has shown inhibitory activity against 5α-reductase type II, it did not activate dermal papilla cells of hair follicles in vitro [Extracts for the Hair Growth Stimulation using In vivo and In vitro Test Models, 2002, oldkmbase.medric.or.kr/KMID/1189420020150020053], This may indicate that 5α-reductase type I is not affected by the extract, as 5α-reductase types I and II can substitute for each other's activity when one of the enzymes is deficient in the body [Ali K., Li W., Wu G. Evolution, classification, structure, and functional diversification of steroid 5α-reductase family in eukaryotes //Heliyon, 2024].

**Table 4. Assessment of the amount of 5α-Reductase Type I in skin sebocytes**

| **Test Sample No.** | **Composition components and their concentration (wt. %)** | | **5α-reductase type I level (mg/mL)** | **Reduction in the amount of 5α-reductase type I relative to the difference between the negative control and the stress factor, %** |
|---|---|---|---|---|
| 1 | negative control (purified water) | | 0.409±0.005 | - |
| 2 | Stress factor: testosterone 10 nM | | 0.640±0.065 | +56.5% |
| 3 | Shikimic acid | 0.03% | 0.484±0.039 | -67.5%* |
| | | 0.1% | 0.445±0.027 | -84.7%** |
| | | 0.3% | 0.407±0.021 | -101.1%*** |
| 4 | Shikimic acid and *Prunus Mume* fruit extract in a 1:1 mass ratio | 0.03% | 0.408±0.072 | **-100.5%***** |
| | | 0.1% | 0.387±0.011 | **-109.9%***** |
| | | 0.3% | 0.365±0.007 | **-119.3%***** |
| | | 1.0% | 0.340±0.003 | **-129.7%***** |
| 5 | Salicylic acid (active control No. 1) | 0.03% | 0.451±0.066 | -82.2%** |
| | | 0.1% | 0.446±0.014 | -84.1%** |
| | | 0.3% | 0.440±0.043 | -86.5%** |
| 6 | Azelaic acid + salicylic acid in a 1:1 mass ratio (active control No. 2) | 0.03% | 0.533±0.022 | -66.9%* |
| | | 0.1% | 0.486±0.045 | -73.8%* |
| | | 0.3% | 0.470±0.003 | -82.1%** |
| Significance levels: *p<0.05; **p<0.01; ***p<0.001 | | | | |

Salicylic acid is widely used for mild acne due to its anti-inflammatory, fungistatic, and bacteriostatic properties [Krautheim A., Gollnick H. Transdermal Penetration of Topical Drugs Used in the Treatment of Acne // Clinical Pharmacokinetics. 2003. V. 42. No. 14]. Salicylic acid does not sufficiently normalize hormonal balance and the activity of 5α-reductase type I in skin cells, resulting in a delayed clinical effect, observed only in cases of acne caused by bacteria. According to in vitro studies, salicylic acid at concentrations ranging from 0.03% to 0.3% has a moderate effect, weakly dependent on concentration: reducing the amount of 5α-reductase type I from 82.2% to 86.5%, which is not comparable to the effect of the combination of shikimic acid and *Prunus Mume* fruit extract according to the invention.

Azelaic acid is also considered an antibacterial agent, possessing anti-inflammatory, antioxidant, and exfoliating properties [Implications of azelaic acid's multiple mechanisms of action: Therapeutic versatility // Journal of the American Academy of Dermatology. 2008. No. 2 (58).]. It has been previously reported that azelaic acid is a potent inhibitor of 5α-reductase: partial inhibition of the enzyme was observed at concentrations as low as 0.2 mmol/L, and complete inhibition was achieved at 3 mmol/L [D. STAMATIADIS et al. Inhibition of 5α-reductase activity in human skin by zinc and azelaic acid //British Journal of Dermatology, 1988, Volume 119, Issue 5, 627-632]. According to in vitro studies, the combination of azelaic acid and salicylic acid in a 1:1 mass ratio at concentrations from 0.03% to 0.3% has a less significant effect than salicylic acid alone. It is believed that azelaic acid and salicylic acid, when present together, reduce each other's efficacy, resulting in no enhancement of the effect or synergy. A reduction in the amount of 5α-reductase type I from 66.9% to 82.1% was observed, which is not comparable to the effect of the combination of shikimic acid and *Prunus Mume* fruit extract according to the invention.

The authors unexpectedly discovered that only the combination of *Prunus Mume* fruit extract and shikimic acid in a 1:1 mass ratio, when used together, demonstrates a statistically significant positive effect on the normalization of hormonal stress and its consequences in skin sebocytes within the claimed concentration range. The authors unexpectedly demonstrated that the combination of *Prunus Mume* fruit extract and shikimic acid in a 1:1 mass ratio reduced the average amount of 5α-reductase type I at various concentrations and exhibited synergy, allowing for the selection of the lowest dosage while maintaining the effect (statistically significant difference compared to the negative control). This effect was more pronounced compared to shikimic acid, salicylic acid, and the combination of salicylic acid with azelaic acid.

Thus, the combination of shikimic acid and *Prunus Mume* fruit extract in a 1:1 mass ratio, when used together, is useful for managing hormonal skin stress and the epigenetic modulation of 5α-reductase type I in skin cells, particularly epidermal cells, sebocytes, and hair follicle cells, and allows for the reduction of the consequences of hormonal stress induced by testosterone and steroid sex hormones related to testosterone.

### Example 5.

An evaluation was conducted on the process applicability of products with the composition according to the invention, specifically thin patches composed of the components listed in Table 1 (Example 1). The production of thin patches from hydrocolloid mass is a complex technological process that requires a special method of preparation and incorporation of the composition. Since shikimic acid and *Prunus Mume* fruit extract are water-soluble substances, and the thin patches contain less than 5% free water for dissolving these substances, this presents a process challenge.

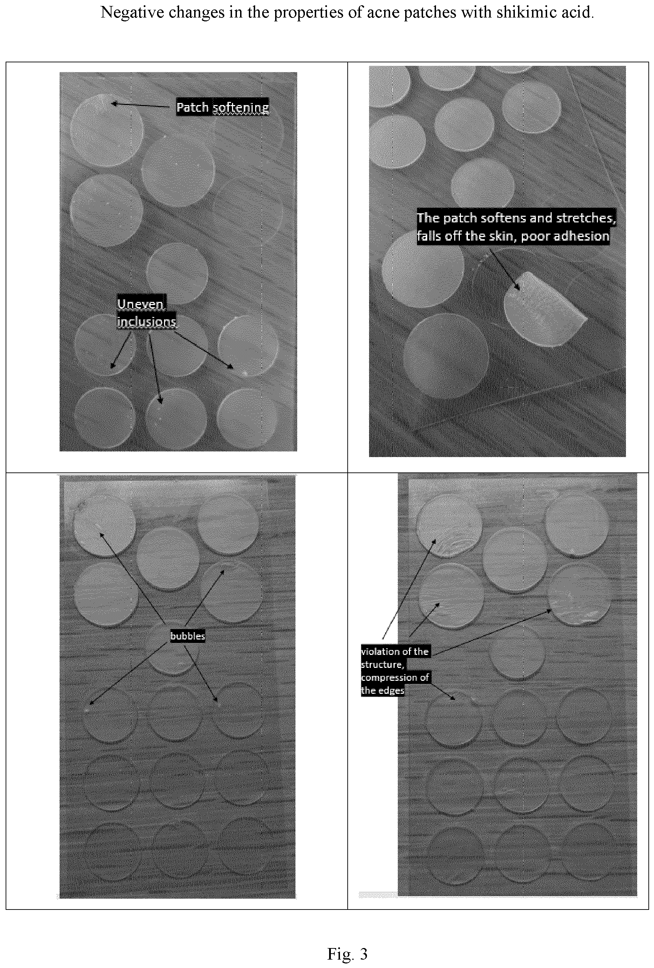

During the production of pilot samples of thin acne patches, it was found that shikimic acid at a concentration of 0.5 wt.% (active working concentration according to the results of the study in Example 4) significantly impaired the stability and appearance of the acne patches. These changes manifested as the crystallization of shikimic acid across the surface of the patches, presumably due to its low solubility in anhydrous media. As a result, the patch samples had an uneven surface, crystalline inclusions, softening, reduced adhesion to the skin surface, discoloration during the stability test at 42+2°C, and disruption of the plasticity of the colloid mass (Figure 3). These changes critically affect the properties of the acne patches, leading to uneven release of shikimic acid into the skin and poor adhesion to the skin, preventing effective delivery of the substance deep into inflamed pores in cases of acne.

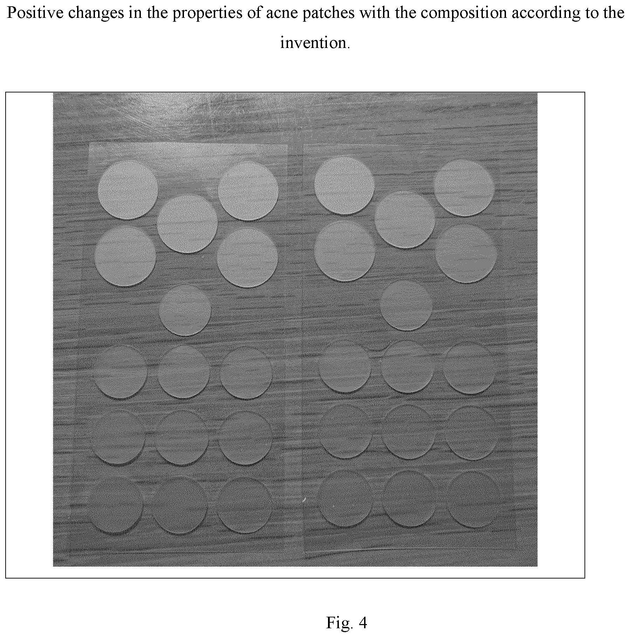

To solve this process problem, the use of solvents (propylene glycol, glycerin, 1,2-hexanediol) did not achieve uniform dissolution of shikimic acid in the patches or ensure their stability over a long shelf life. However, the authors unexpectedly discovered that *Prunus Mume* fruit extract can enhance the solubility of shikimic acid in anhydrous media, stabilize it, and prevent crystallization by forming a self-dissolving (plant eutectic) mixture. The use of a combination of shikimic acid and *Prunus Mume* fruit extract in a 1:1 mass ratio and a total concentration of 1 wt.% allowed for mutual dissolution in a nearly anhydrous hydrophobic hydrocolloid mass without crystallization and without loss of the elastic-viscous-plastic properties of the acne patches. These patches exhibited improved adhesion, stability, and an absence of inclusions, unevenness, air bubbles, or crystals across the surface (Figure 4).

To preserve the consumer and mechanical properties of the acne patches, it is necessary to first prepare a premix of shikimic acid in *Prunus Mume* fruit extract in a 1:1 mass ratio, with gradual addition of shikimic acid and stirring on a magnetic stirrer at a speed of at least 300 rpm for no less than 20 minutes. Shikimic acid begins to dissolve in the *Prunus Mume* fruit extract due to the high content of amphiphilic and lipophilic compounds, particularly flavonoids, which stabilize shikimic acid through a related chemical structure. After preparing the premix, it is introduced into the final hydrocolloid mass without heating, with uniform mixing until the premix of the composition according to the invention is completely dissolved and incorporated.

Thus, the composition according to the invention, based on shikimic acid in *Prunus Mume* fruit extract in a 1:1 mass ratio, exhibits improved process applicability and stability in acne patches for subsequent use in the product and ensures clinical efficacy in treating acne-prone skin. The authors unexpectedly discovered that the use of *Prunus Mume* fruit extract improves the solubility and stabilizes shikimic acid when used in low-water-content hydrophobic media, such as the hydrocolloid mass of acne patches, thereby ensuring the consumer properties of the finished product and resolving process incompatibilities.

### Example 6.

A clinical study was conducted to evaluate the effectiveness of products containing the composition according to the invention in volunteers with mild to moderate acne. Thin patches composed of the components listed in Table 1 (Example 1) were used as the base for incorporating the components. Thin patches containing 0.5% encapsulated salicylic acid as the main active ingredient for the prevention and treatment of acne were selected as a positive control.

To determine the claimed effects of the thin acne patches containing the composition according to the invention, a prospective open non-randomized single-center controlled study was conducted on 15 male and female volunteers aged 17 to 28 years, with an average age of 19.8 years, who had oily or combination skin. To assess the effectiveness of the acne patches, the group of volunteers used the product according to their individual personal hygiene routines and in accordance with the manufacturer's recommendations (determine the size of the skin inflammation, cleanse the skin in the usual way, apply the patch to the inflamed area, evaluate the area of inflammation after 6-8 hours, and remove the patch if a positive result was achieved). The volunteers met the inclusion criteria and had no exclusion criteria. The study was conducted in accordance with the Declaration of Helsinki and after signing informed consent for voluntary participation in the study.

Before the start of the study, an assessment of sensitizing and irritating properties was conducted by a single application of the product under study with the composition according to the invention on the skin under the supervision of a dermatologist. Additionally, an evaluation of the functional parameters of the skin was performed - trans-epidermal water loss (TEWL), sebumetry, comedogenicity, erythema and pigmentation indicators when using the products. Before the study began, all subjects underwent a preliminary examination, including a physical examination, analysis of concomitant diseases, and a thorough collection of dermatological, allergological, and pharmacological history. All studies were conducted under the same conditions of humidity and air temperature of 22±2°C and relative humidity of 40-60%. The effects were evaluated using certified medical equipment: Cutometer^{®} 580 MPA, equipped with a built-in Sebumeter^{®} SM 815 sensor (sebumetry); mexameter with a special lamp with 2 wavelengths (mexametry); Tewameter^{®} TM 300 (tewametry); and a UV lamp with a magnifying glass from "IONTO-COMED GmbH" (Germany).

The clinical study included several visits to the dermatologist:
1. Visit 0: Informed consent was obtained, and a visual assessment of the skin condition was conducted using instrumental diagnostic methods. After this, thin patches were applied to selected areas with early inflammations.
2. Visit 1: 8 hours after the start of the study and the application of the patch to the inflamed area. The researcher-physician removed the patches, and then the volunteer rested for 10 minutes to exclude any false reaction to the patch adhesive. Then the researcher-physician conducted a clinical examination, assessed the local status, and performed a visual inspection. If the early inflammation area did not resolve within 8 hours, the researcher-physician measured the skin parameters and applied new thin patches.
3. Visit 2: 24 hours after the first application of the product under study. The researcher-physician removed the patches, and then the volunteer rested for 10 minutes to exclude any false reaction to the patch adhesive. Then the researcher-physician conducted a clinical examination, assessed the local status, and performed a visual inspection. If the early inflammation area did not resolve within 24 hours, the researcher-physician measured the skin parameters and applied new thin patches.
4. Visit 3: 48 hours after the first application of the product under study. The researcher-physician removed the patches, and then the volunteer rested for 10 minutes to exclude any false reaction to the patch adhesive. Then the researcher-physician conducted a clinical examination, assessed the local status, and performed a visual inspection. If the early inflammation area did not resolve within 48 hours, the researcher-physician measured the skin parameters and made a note in the patient's individual medical record.

For all quantitative data, the group arithmetic mean (M), median (Me), and standard deviation (SD) were calculated. Variables such as proportion of volunteers were assessed descriptively as percentages. Data on parameters were presented based on the nature of the variable, whether continuous or categorical. Data for categorical variables were analyzed as the number (n) and percentage of the total number of volunteers with a given value of the characteristic being considered. The results obtained were processed using the Statistica package (StatSoft, USA, Ver. 8.0).

### Results.

During the trial, involving the use of the products under study throughout the entire clinical study period, including home use, no serious adverse events or adverse reactions were reported among the participants, indicating good tolerability of the products under study. At the final stage (Visit 2), 48 hours after the start of the study, the physician's visual examination of the volunteers revealed positive dynamics in the condition of the skin in the vast majority of cases - elimination or resolution of early inflammatory elements, reduction of inflammation and redness, alleviation of subjective discomfort, and evening of skin tone. Additionally, there was no recurrence of inflammatory processes at the sites of patch application, no pore blockage, and no worsening of the initial skin condition.

The effect of eliminating and controlling acne at an early stage within 8-24 hours was achieved in 73% of volunteers in the group (11 out of 15 individuals). In 53% of volunteers who used the thin patches with the composition based on shikimic acid and *Prunus Mume* fruit extract according to the invention, complete resolution of early inflammatory elements, regression of subjective sensations, and visible improvement in skin condition were observed within 8 hours, indicating a rapid effect and normalization of hormonal stress in cases of mild acne. In 20% of volunteers, complete elimination of early inflammatory elements and regression of subjective sensations occurred within 24 hours. In the remaining 27% of volunteers, the inflammatory elements resolved within 48 hours. The dermatologist noted the elimination of early inflammations with high efficacy and a safe impact on the skin. Early-stage pimples disappeared within 24 hours, while itching and redness were significantly reduced after one application for 8 hours (Table 5).

In the case of the positive control - patches containing 0.5% salicylic acid - the effect of eliminating and controlling acne at an early stage within 8-24 hours was achieved in 73% of volunteers, but with a predominance of cases after 24 hours of continuous patch contact with the skin. In 33% of volunteers who used the thin patches with salicylic acid, complete resolution of early inflammatory elements, regression of subjective sensations, and visible improvement in skin condition were observed within 8 hours. In 40% of volunteers, complete resolution of early inflammatory elements and regression of subjective sensations occurred within 24 hours. In the remaining 27% of volunteers, the inflammatory elements resolved within 48 hours. Since salicylic acid is an antibacterial component and does not regulate the activity of 5α-reductase type I in the skin under hormonal stress, a significantly longer time was required to achieve a clinically significant effect in the majority of volunteers, which is a disadvantage of using such a product for quickly controlling acne.

**Table 5. Dynamics of clinical and subjective symptoms of acne after using patches with active ingredients**

| **Product Description** | **Number of volunteers with improvements after visual and instrumental diagnosis by a dermatologist, abs.% (N=15)** | |
|---|---|---|
| | Thin patches with the composition according to the invention (0.5% shikimic acid and 0.5% *Prunus Mume* fruit extract, mass ratio 1:1) | Thin patches with 0.5% salicylic acid (positive control) |
| Complete reduction of redness, erythema | | |
| **Visit 0 before use** | - | - |
| **Visit 1 after use (8 hours)** | 7 | **5** |
| **Visit 2 after use (24 hours)** | 11 | 11 |
| **Visit 3 after use (48 hours)** | 15 | 15 |

| Reduction of itching and painful sensations | | |
|---|---|---|
| **Visit 0 before use** | - | - |
| **Visit 1 after use (8 hours)** | **11** | **5** |
| **Visit 2 after use (24 hours)** | 15 | 11 |
| **Visit 3 after use (48 hours)** | 15 | 15 |

According to the results of the dynamic study of functional indicators in the group of volunteers using biophysical research methods, significant improvement in the physiological parameters of the skin was observed when using the thin patches with the composition according to the invention (Table 6) compared to the thin patches with salicylic acid. The combination of shikimic acid and *Prunus Mume* fruit extract in a 1:1 mass ratio significantly reduced skin sebum content within 48 hours of using the thin patches, which helps normalize the physiological state of the inflammation area in acne and reduce the impact of sebocytes on acne pathogenesis. Since skin sebum and its excessive amount are among the main triggering factors in addition to bacteria and hormonal imbalance of the skin (activity of 5α-reductase type I and dihydrotestosterone content), the normalization of this parameter and the negative dynamics confirm the improvement in skin condition. Patches with salicylic acid also have a moderate sebum-regulating effect but are less effective than patches with the combination according to the invention. This fact is confirmed by the results of the in vitro study on the regulation of 5α-reductase type I levels in skin sebocytes, where the combination of shikimic acid and *Prunus Mume* fruit extract in a 1:1 mass ratio significantly reduced the amount of 5α-reductase type I in skin sebocytes under the inflammatory hormonal stress of testosterone more than salicylic acid and the combination of salicylic and shikimic acids (Example 4, Table 4).

**Table 6. Assessment of skin sebum content during the clinical study**

| **Product Description** | **Skin sebum content, ng/ cm² (n=15)** | | |
|---|---|---|---|
| | **Visit 0 before use** | **Visit 2 after 48 hours of use** | **Change** |
| Thin patches with the composition according to the invention (0.5% shikimic acid and 0.5% *Prunus Mume* fruit extract, mass ratio 1:1) | 245.3 | 205.6 | -16.18% |
| Thin patches with 0.5% salicylic acid (positive control) | 237.5 | 211.6 | -10.91% |

The combination of shikimic acid and *Prunus Mume* fruit extract in a 1:1 mass ratio in the thin patches significantly reduced transepidermal water loss (TEWL) after 8 and 48 hours, helping to normalize the condition and integrity of the epidermis at the site of patch application. In cases of mild to moderate acne, the inflammatory zone often shows a compromised barrier due to the active inflammatory response, swelling, and pain, leading to increased TEWL and exacerbating itching, sensitivity, and erythema. A 24.4% reduction in TEWL in the test group of volunteers indicates significant improvement in skin condition. Patches with salicylic acid exhibit a more moderate moisture-regulating effect, which is less pronounced than that of the patches with the combination according to the invention. Improving the skin condition in the inflamed area is one of the main factors in skin health and acne prevention. The active composition according to the invention provides this effect in the context of hormonal stress such as testosterone, demonstrating the resolution of inflammatory skin manifestations in acne among young volunteers (Table 7).

**Table 7. Assessment of transepidermal water loss (TEWL) during the clinical study**

| **Product Description** | **TEWL Index, g/h*m ² (n=15)** | | |
|---|---|---|---|
| | **Visit 0 before use** | **Visit 2 after 48 hours of use** | **Change** |
| Volunteers with improvements in inflammatory acne manifestations after 8 hours | | | |
| Thin patches with the composition according to the invention (0.5% shikimic acid and 0.5% *Prunus Mume* fruit extract, mass ratio 1:1) | 16.8 | 12.7 | -24.4% |
| Thin patches with 0.5% salicylic acid (positive control) | 16.6 | 13.7 | -17.5% |

| Volunteers with improvements in inflammatory acne manifestations after 48 hours | | | |
|---|---|---|---|
| Thin patches with the composition according to the invention (0.5% shikimic acid and 0.5% *Prunus Mume* fruit extract, mass ratio 1:1) | 20.1 | 16.5 | -17.9% |
| Thin patches with 0.5% salicylic acid (positive control) | 20.6 | 17.3 | -16.0% |

14 out of 15 volunteers (93%) reported high efficacy of the thin patches with the composition based on shikimic acid and *Prunus Mume* fruit extract and the elimination of early inflammatory manifestations, as per the questionnaire results. In contrast, only 12 out of 15 volunteers (80%) reported high efficacy of the salicylic acid patches and the elimination of early inflammations, which may be insufficient in cases of rapidly developing skin processes and active hormonal stress with inflammation. All 15 volunteers (100%) noted the prevention of post-acne formation, reduction of inflammation, evening of skin tone, and visual improvement in skin condition when using thin patches with the composition based on shikimic acid and *Prunus Mume* fruit extract at all stages of the clinical study.

According to the test results, the composition based on shikimic acid and Prunus Mume fruit extract reduced the effects of hormonal stress induced by testosterone, cortisol, and steroid sex hormones, epigenetically modulated the quantity and expression of 5α-reductase type I in various skin cells. These molecular changes are corroborated by clinical study data.

## Claims

1. The use of
A) shikimic acid or a salt thereof, and
B) an extract of *Prunus Mume,*
or the use of a kit, kit-of-parts comprising said both components A and B,
in the manufacture of a skin care and/or skin cleaning composition, wherein the mass ratio of said components A and B in terms of active ingredients in said composition is (0.10-1.00):(0.001-1.00) wt. %.

2. The use of claim 1, wherein
i) said composition comprises component A and component B in a ratio of 1:1 and/or
ii) said components A and B are comprised by said composition in an amount of 0.10 wt.
%, 0.15 wt. %, 0.20 wt. %, 0.25 wt. %, 0.30 wt. %, 0.35 wt. %, 0.40 wt. %, 0.45 wt.
%, 0.50 wt. %, 0.55 wt. %, 0.60 wt. %, 0.65 wt. %, 0.70 wt. %, 0.75 wt. %, 0.80 wt.
%, 0.85 wt. %, 0.90 wt. %, 0.95 wt. %, 1.00 wt. %, or any values in between; or any ranges based on any combination of said values
and /or
iii) said composition further comprises exosomes and *Lespedeza cuneata* extract.

3. The use of claim 1 or claim 2, wherein said shikimic acid has the CAS 138-59-0 registration number.

4. A composition comprising
A) shikimic acid or a salt thereof, and
B) an extract of *Prunus Mume,*
wherein the mass ratio of said components A and B in terms of active ingredients in said composition is (0.1-1.0):(0.001-1.0) wt. %.

5. The composition of claim 4, wherein
i) said composition comprises component A and component B in a ratio of 1:1 and/or
ii) said components A and B are comprised by said composition in an amount of 0.10 wt.
%, 0.15 wt. %, 0.20 wt. %, 0.25 wt. %, 0.30 wt. %, 0.35 wt. %, 0.40 wt. %, 0.45 wt.
%, 0.50 wt. %, 0.55 wt. %, 0.60 wt. %, 0.65 wt. %, 0.70 wt. %, 0.75 wt. %, 0.80 wt.
%, 0.85 wt. %, 0.90 wt. %, 0.95 wt. %, 1.00 wt. %, or any values in between; or any ranges based on any combination of said values.
and /or
iii) said composition further comprises exosomes and *Lespedeza cuneata* extract.

6. The composition of claim 4 or claim 5, wherein said shikimic acid has the CAS 138-59-0 registration number.

7. The composition of any one of claims 4-6, wherein said composition is a topical formulation and/or is formulated for application on a subject's skin.

8. The composition of any one of claims 4-7, wherein said composition is a formulation selected from the following:
- film;
- liquid;
- aerosol;
- suspension,
- solution,
- tincture,
- cream,
- paste,
- lotion,
- ointment,
- gel,
- balsam,
- foam,
- powder.

9. The composition of any one of claims 4-8, wherein said composition is a skin care and/or skin cleaning composition.

10. The composition of any of claims 4-9, wherein said composition is a pharmaceutical composition.

11. The composition of any of claims 4-10, wherein said composition is for use as a medicament.

12. The composition of any of claims 4-11, wherein said composition is for use in the treatment of a subject suffering from at least one of the following diseases or symptoms:
- inflammation of the skin;
- acne and/or pimple;
- dermatitis, including seborrheic and atopic dermatitis;
- psoriasis;
- eczema;
- vitiligo;
- xerosis;
- xerostomia;
- hyperkeratosis, including follicular hyperkeratosis;
- rosacea;
- keratolysis.

13. Non-medical use of any one of the composition of claims 4-9, wherein said non-medical use of said composition is preferably in cosmetology.

14. The non-medical use of claim 13, wherein said composition is as a component of a body care product, wherein said body care product is preferably selected from the group consisting of
- a cosmetic and/or skin cleanser, further preferably a facial skin cleanser;
- a mask and/or patch, including a cleansing mask or patch comprising said composition, preferably for application to a subject's face, neck, trunk, and arms.

15. A method of manufacturing a patch from an hydrocolloidal media comprising less than 5 wt.% water and comprising the composition of claim 4, and said composition preferably comprising components A and B in an amount of 0.50 wt.%, wherein said method comprises:
- pre-mixing said shikimic acid in said extract of *Prunus Mume* in a mass ratio of 1:1 with gradual addition of said shikimic acid to said extract of *Prunus Mume* by stirring on a magnetic stirrer at a speed of at least 300 rpm for at least 20 minutes;
- adding said pre-mix to said hydrocolloid media without heating under uniform stirring until said premix is completely dissolved, and
- forming a patch therefrom.
